# EUROPEAN PATENT SPECIFICATION

(11) **EP 0 676 201 B1**
(45) Date of publication and mention of the grant of the patent: **11.09.2002**
(21) Application number: 95301772.0
(22) Date of filing: 16.03.1995
(51) Int. Cl.: A61K 31/498, A61P 35/00, C07D 241/46

(54) **Use of a riminophenazine for treating MDR resistance**
Verwendung von Riminophenazin zur Behandlung von MDR-Resistenz
Emploi d'une riminophénazine dans le traitement de la résistance MDR

(30) Priority: 05.04.1994 ZA 942363; 24.11.1994 ZA 949352
(43) Date of publication of application: 11.10.1995
(73) Proprietor: Universiteit van Pretoria, 0002 Gauteng (ZA)
(72) Inventor: Medlen, Constance Elizabeth, Rietondale, Pretoria 0084 (ZA); Anderson, Ronald, The Willows, Pretoria 0041 (ZA); O'Sullivan, John Francis, Blackrock, Co. Dublic (IE)
(74) Representative: Ouzman, Beverley Nicola Claire

(56) References cited:
- INT.J.LEPR.OTHER MYCOBACT.DIS., vol. 61, no. 3, 1993, pages 406-14, XP000568242 "Activity of phenazine analogs against mycobacterium leprae infections in mice"
- TUBERCLE AND LUNG DISEASE, vol. 75, no. Suppl.1, 17 June 1994, pages 25-26, XP000568247 "Activity of riminophenazine compounds against susceptible and drug resistant M.Tuberculosis"
- CANCER LETT., vol. 85, no. 1, 30 September 1994, pages 59-63, XP000568244 "The riminophenazine agents clofazimine and B699 reverse aquired multidrug resistance in a human lung cancer cell line"
- INT.J.LEPR.OTHER MYCOBACT.DIS., vol. 34, no. 4, 1966, page 391-7 XP000568332 "Studies on sulfone resistance in leprosy. Treatment with a riminophenazine derivative (B663)"
- BR.J.DIS.CHEST, vol. 64, no. 3, 1970, pages 161-3, XP000568330 "Drug-resistant Pulmonary Tuberculosis treated with ethambutol, a rifamycin, and a riminophenazine (B663)"
- J.MED.CHEM., vol. 31, no. 3, 1988, pages 567-72, XP002000457 "Clofazimine analogs active against a clofazimine-resistant organism"

## Description

### BACKGROUND TO THE INVENTION

THIS INVENTION relates to substances or compositions for use in therapeutic treatments. More particularly, the invention relates to patients who have built up, or could build up, resistance to therapeutically active substances, to novel compounds for use therein, to therapeutic compositions and their use, as well as to methods of preparing such compounds and compositions and methods of use thereof.

A problem in the therapy of diseases which attack cells of the body is that the cells become resistant to drugs used for treating them. For example, in the treatment of cancer, several drugs are often used and the cells become resistant to these drugs. In the case of the chemotherapeutic treatment of tumours, multi-drug resistance mechanisms become operative and render the cells resistant to many treatment agents. The multi-drug resistance may be intrinsic or may be acquired. When it is acquired, the resistance results in relapse after an initially favourable response, and can be mediated by P-glycoprotein, which is an energy dependent multi-drug afflux pump and the product of the MDR 1 gene. As a result of the acquired multi-drug resistance mechanisms, the P-glycoprotein tends to pump out further amounts of drug being utilized and thereby prevents the therapeutic effect of such drugs taking place on the cells. Similarly, in the radiotherapeutic treatment of cancer, P-glycoprotein can be induced by such treatment, with the result that cell resistance to further cancer treatment can result.

Such a mechanism of multi-drug resistance is thus operative in cancer patients who develop acquired drug resistance during chemotherapy or radiotherapy, thereby resulting in a pattern of resistance to a wide variety of anti-cancer drugs such as vinca alkaloids, epipodophyllotoxines, actinomycin D, anthracyclines, mithramicin, taxol, and the like. Accordingly, if one can discover substances or compositions which are able to modulate P-glycoprotein, continued treatment of the patient with the therapeutic drugs becomes possible. In this connection, we are aware that cyclosporin A has the property of acting as a multi-drug afflux pump modulator and thereby enabling treatment drugs to remain in the cells and provide a therapeutic effect.

We have now surprisingly found that riminophenazines possess multi-drug resistance activity (MDR activity) and the invention includes compounds and compositions for such use as well as novel riminophenazines.

### DESCRIPTION OF THE PRIOR ART

Many riminophenazines are known. Also, therapeutic activity for many riminophenazines is known. The known therapeutic activity usually has been for use in the treatment of tuberculosis or leprosy.

The riminophenazines are compounds having a phenazine ring which are substituted on a ring nitrogen and which contain substituents in one or both of the fused benzene rings, one of said substituents being a substituted or unsubstituted imino group. The substituent on the imino group appears to be important in defining the activity, as do a substituted amino group in the same benzene ring and ring the nitrogen substituent.

For example, in a series of U.S.A. patent specifications, namely nos. 2,943,089; 2,946,792 and 2,948,726, Barry *et al* described a number of riminophenazines which had activity against tubercula bacillus and which were stated to be tubercutaostatic. Two of the substituted positions, namely the amino group in the 2-position, the imino group in the 3-position and the nitrogen atom in the 5-position were substituted by phenyl or substituted phenyl groups and the other substituent was a dialkylaminoalkyl, alkoxyphenyl, alkyl or cycloalkyl radical. One such compound is clofamazine, N,5-*bis-*(4-chlorophenyl)-3,5-dihydro-3-[1-methylethyl)imino]-2-phenazinamine.

More recently, many hundreds of riminophenazines have been made with various phenyl or substituted phenyl substituents on the 2-amino and 5-nitrogen positions and with the 3-imino radical having a variety of substituents thereon. Pharmaceutical properties have been found for a number of these known riminophenazines.

None of these prior art compounds has been reported to have MDR activity.

### DESCRIPTION OF THE INVENTION

In one aspect, the invention provides the use of a riminophenazine in the manufacture of a medicament to treat a cancer patient who has built up, or could built up, resistance to a therapeutically active substance used in the treatment of cancer. In a second aspect of the invention, the invention provides novel riminophenazines, their use, and a method for their preparation.

In a third aspect, the invention provides a composition for the treatment of a cancer patient who has built up, or could build up, resistance to a therapeutically active compound used in the treatment of cancer, said composition containing a novel riminophenazine and a pharmaceutically acceptable carrier.

In a fourth aspect, the invention provides a pharmaceutical composition comprising a novel riminophenazine, a known cancer treatment compound and a pharmaceutically acceptable carrier.

A riminophenazine is a phenazine containing a substituent on a ring nitrogen atom and a substituted or unsubstituent imino substituent in one of the benzene rings. The imino group conveniently may be in the 2- or 3- position, the nitrogen atoms of the phenazine being in the 5- and 10- positions. Conveniently, there may also be an ammo group in the same benzene ring as the imino group, preferably in the 3- or 2-position. A presently preferred riminophenazine may have a 2-(substituted amino)-3-(substituted imino)-5-aryl grouping, optionally with a further substituent in the 8-position, i.e. a compound of the general formula with appropriate substituents on the free bonds shown:

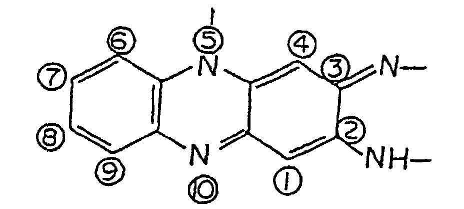

One preferred group of riminophenazines for use in the first aspect of the invention are compounds of general formula (I), 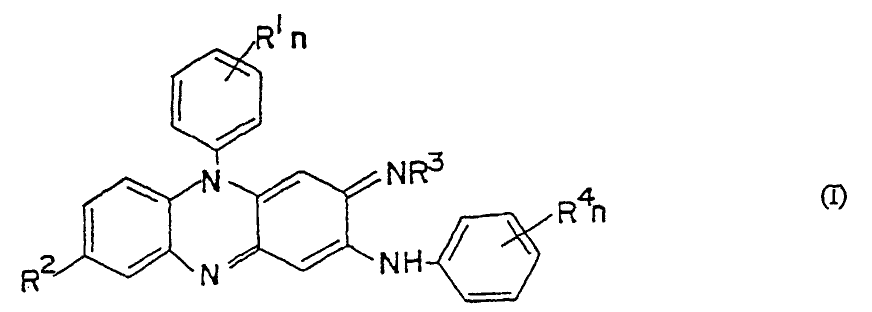 in which
R¹ and R⁴ are the same and each is a hydrogen atom, a halogen atom, or an alkyl radical,
R² is a hydrogen or halogen atom,
R³ is selected from hydrogen, alkyl, substituted alkyl, cycloalkyl, cycloalkylalkyl or is a heterocyclic or heterocyclic-alkyl radical,
n is 1, 2 or 3.

The riminophenazines act on cells of the human or animal body to make them susceptible to the action, or continued action, of the therapeutically effective compound. This can be particularly important where a patient has been treated repeatedly with a therapeutically effective compound and has built up resistance to that compound, e.g. during cancer treatment.

The radicals R¹ and R⁴ in formula (I) may, for example be hydrogen, chlorine, methyl, isopropyl. R¹ may conveniently be in the 3- and/or 4- position. R² may conveniently be hydrogen or chlorine.

The radical R³ in the above formula (I) may for example, be hydrogen, C₁ - C₄ - lower alkyl, (e.g. methyl, ethyl, n-propyl or iso-propyl), cyclopropyl, cyclobutyl, cyclopentyl, cyclohexyl, cycloheptyl, methylcyclohexyl, hydroxycyclohexyl,cyclooctyl, cyclododecyl, dialkylaminoalkyl, cyclohexylmethyl, piperidyl, alkyl-substituted piperidyl or benzyl substituted pyridyl.

A particularly convenient radical R³ in the above formula (I) is a tetramethylpiperidyl (TMP) radical, e.g. a 4-TMP radical, or a cyclohexyl or a N,N-diethyl- aminopropyl radical.

The known riminophenazines may be prepared by methods described in the literature, e.g. in the prior art including that referred to above.

The invention particularly provides the use in the manufacture of a medicament to treat a cancer patient who has built up or could build up, resistance to a therapeutically active substance used in the treatment of cancer of a novel riminophenazine of the aforementioned general formula (I), in which:
R¹ and R⁴ are the same and each is a halogen atom or a (C₁-C₄) lower alkyl, (C₁-C₄)-alkoxy, (C₁-C₄) fluoroalkyoxy or haloalkyl, (for example trifluoromethyl) radical,
R² is a hydrogen or halogen atom,
R³ is a 4-tetramethylpiperidyl radical
n is 1, 2 or 3, with the provisos that (a) when n is 2 or 3, (R¹)n and (R⁴)n are not 3,4-dichloro and that (b) when n is 1, substituents R¹ and R⁴ cannot represent (C₁-C₄)-alkyl or (C₁-C₄)-alkoxy radicals or a halogen in the 4-position.

The invention provides such compounds *per se,* their preparation and their use as pharmaceutically useful compounds in MDR treatment.

Subject to provisos (a) and (b), the radicals R¹ and R⁴ in the novel compounds may, for example, be chlorine, methyl, isopropyl, methoxy, trifluoromethoxy or trifluonomethyl, R⁴ may conveniently be in the 3- and/or 4-position, R² may conveniently be hydrogen or methyl and R³ preferably is the 4-TMP radical.

The riminophenazines of the above general formula (I) may be prepared from a 1-anilino-2-nitrobenzene.

For example a 1-anilino-2-nitrobenzene of general formula (II) may be reduced, e.g. with hydrogen in the presence of a palladium carbon catalyst, or in zinc and acetic acid, to form the corresponding 1-anilino-2-amino benzene (i.e. 2-amino-diphenylamine) of general formula (III), in which R¹, R² and n have the meanings defined above. Heating at temperatures of 40-55°C can be used.

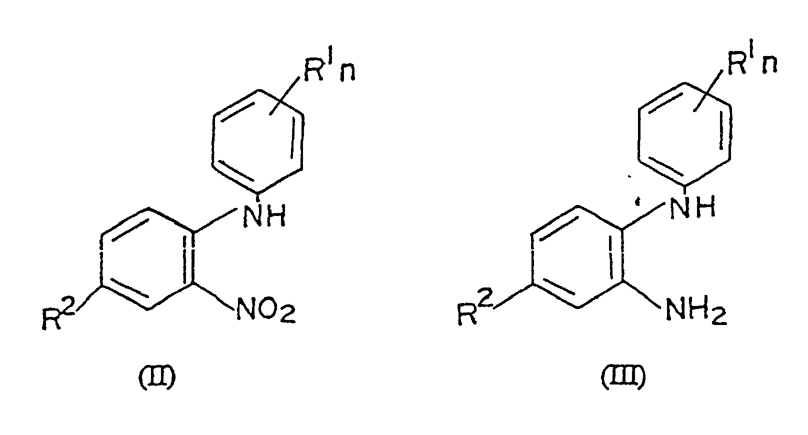

The diphenylamine of formula (III) may be oxidatively condensed, e.g. with ferric chloride and concentrated hydrochloric acid or acetic acid to form a riminophenazine of general formula (IV), i.e. a compound of formula (I) in which R³ is hydrogen. Ethyl alcohol may be used as a solvent. Stirring at ambient temperatures of, preferably, below 15°C may be carried out.

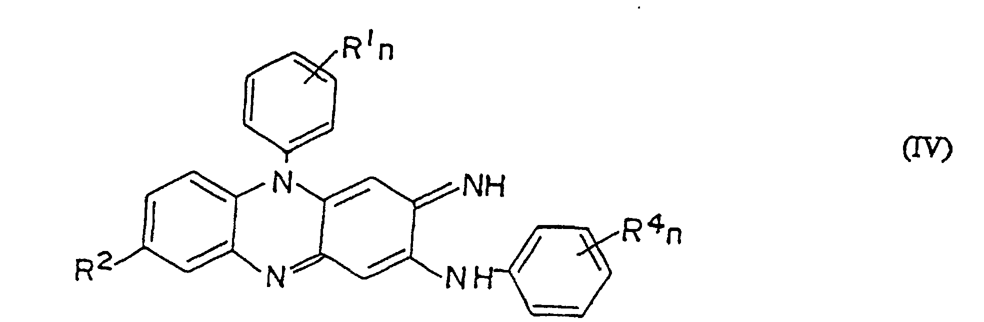

The other riminophenazines of general formula (I), i.e. those which have R³ equal to other than hydrogen, can be formed from the riminophenazine of general formula (IV) by reaction with an amine of formula R³-NH₂. Refluxing of the reactants, in solution in dioxane, for a period of 3 to 5 hours may be necessary.

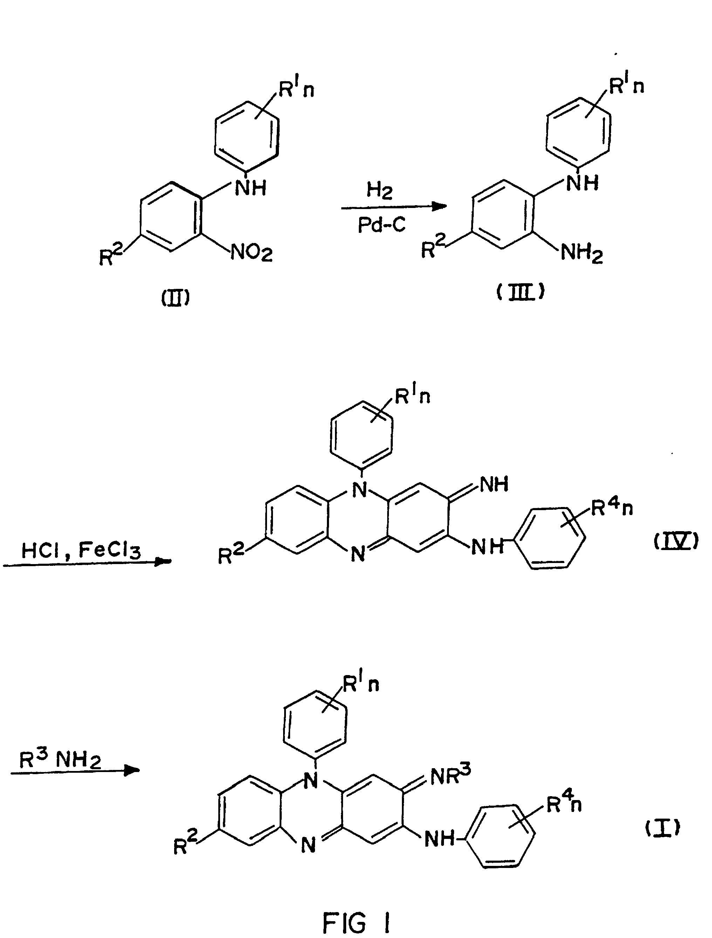

The overall reaction steps are shown in figure 1 of the accompanying drawings.

The 1-anilino-2-nitrobenzene starting material of general formula (II) may be prepared by reacting a 2-halo-nitrobenzene containing a R²-radical in the 5-position, with a formylated aniline having a R¹ substituent in the phenyl ring. The reaction may be carried out in the presence of anhydrous potassium carbonate and while boiling the reactants in dimethylformamide.

The novel compounds of general formula (I) are particularly suitable in the treatment of human or animal cells to make them susceptible to the action or continued action of a therapeutically effective compound. They may be used as the only active compound with one or more acceptable carriers in a composition, The composition may also contains a known cancer treatment compound which is not a riminophenazine.

Particular examples of compounds of general formula (I) are set out in the following Table I:

**TABLE - 1**

| Compound | R¹ | R² | R³ | R⁴ |
|---|---|---|---|---|
| B283 | H | H | H | H |
| B628 | 4-Cl | H | H | H |
| Clofazimine (B663) | 4-Cl | H | -CH(CH₃)₂ | 4-Cl |
| B669 | H | H | -Cyclohexyl | H |
| B670 | H | H | -CH(CH₃)₂ | H |
| B673 | 4-Cl | H | Cyclohexyl | 4-Cl |
| B718 | H | H | -C₂H₅ | H |
| B729 | H | H | Cycloheptyl | H |
| B741 | 4-Cl | H | 4-methylcyclohexyl | 4-Cl |
| B746 | 4-Cl | H | -C₂H₅ | 4-Cl |
| B749 | 4-Cl | H | -(CH₂)₂N.(C₂H₅)₂ | 4-Cl |
| B759 | 4-Cl | H | -(CH₂)₃CH₃ | 4-Cl |
| B796 | H | H | -cyclopentyl | H |
| B980 | 4-F | H | -CH(CH₃)₂ | 4-F |
| B1865 | H | Cl | -CH(CH₃)₂ | H |
| B1912 | H | Cl | -cyclohexyl | H |
| B3677 | 4-me | H | -cyclohexyl | 4-me |
| B3763 | H | H | -cyclohexylmethyl | H |
| B3779 | 4-Cl | H | 4-(N,N-diethylamino)-2-methyl-butyl | 4-Cl |
| B3786 | Cl | H | 4'-TMP | Cl |
| B3825 | 4-Cl | H | 4-hydroxycyclohexyl | 4-Cl |
| B3962 | H | H | 4'-TMP | H |
| B4019 | H | Cl | 4'-TMP | H |
| B4021 | H | Cl | -C₂H₅ | H |
| B4070 | 4-me | H | -4-piperidyl | 4-me |
| B4090 | 4-Cl | Cl | 4'-TMP | 4-Cl |
| B4100 | 3,4-di-Cl | H | 4'-TMP | 3,4-di-Cl |
| B4103 | 4-CF₃ | H | 4'-TMP | 4-CF₃ |
| B4104 | 4-Cl | Cl | Cyclohexyl | 4-Cl |
| B4121 | 3,5-di-Cl | H | 4'-TMP | 3,5-di-Cl |
| B4123 | 3-Cl | Cl | 4'-TMP | 3-Cl |
| B4126 | 3-CF₃ | H | 4'-TMP | 3-CF₃ |
| B4127 | 3-CF₃ | Cl | 4'-TMP | 3-CF₃ |
| B4128 | 2,4-di-Cl | H | 4'-TMP | 2,4-di-Cl |
| B4154 . | 3,4-di-Cl | H | -(CH₂)₃N.(C₂H₅)₂ | 3,4-di-Cl |
| B4158 | 4-CH(CH₃)₂ | H | 4'-TMP | 4-CH(CH₃)₂ |
| B4159 | 4-CH(CH₃)₂ | Cl | 4'-TMP | 4-CH(CH₃)₂ |
| B4163 | 3-CF₃-4-Cl | H | 4'-TMP | 3-CF₃-4-Cl |
| B4166 | H | H | -cyclooctyl | H |
| B4169 | 3,4,5-tri-Cl | H | 4'-TMP | 3,4,5-tri-Cl |
| B4170 | H | H | -cyclopropyl | H |
| B4171 | H | H | -cyclododecyl | H |
| B4172 | H | H | -cyclobutyl | H |
| B4173 | H | H | 4'-(N-benzylpiperidyl) | H |
| B4174 | 4-OCH₃ | H | 4'-TMP | 4-OCH₃ |
| B4175 | 3,4-di-Cl | H | Cyclohexyl | 3,4-di-Cl |
| B4177 | 4-OCF₃ | H | 4'-TMP | 4-OCF₃ |

In the above table, when R³ is 4'-TMP, the TMP radical is a 4-(2,2,6,6,-tetramethyl piperidyl) radical. The abbreviation "me" has been used for methyl.

Presently preferred compounds are those identified as B4103, B4158 and B4169, in view of their good bio-availability and high MDR effects.

The invention also provides those compounds in Table 1 which are believed to be new compounds, for example the compounds B3779, B4070, B4103, B4104, B4121 B4123, B4126, B4127, B4128, B4154, B4158, B4159, B4163, B4166, B4169, B4170, B4171, B4172, B4173, B4174, B4175 and B4177.

The chemical names for certain of the compounds of Table I are set out in Table II below :

**TABLE II**

| | |
|---|---|
| B663 | N,5-*bis*-(4-chlorophenyl)-3,5-dihydro-3-[(1-methylethyl)imino]-2-phenazinamine; |
| B796 | N,5-*bis*-phenyl-3,5-dihydro-3-(cyclopentylimino)-2-phenazinamine; |
| B3677 | N,5-*bis*(4-methylphenyl)-3,5-dihydro-3-(cyclohexylimino)-2-phenazinamine; |
| B3763 | N,5-*bis*(phenyl)-3,5-dihydro-3-[(cyclohexylmethyl)imino]-2-phenazinamine; |
| B3779 | N,5-*bis*(4-chlorophenyl)-3,5-dihydro-3-[(4-diethylamino-2-methylbutyl)imino]-2-phenazinamine; |
| B3962 | N,5-*bis*(phenyl)-3,5-dihydro-3-[(2',2',6',6'-tetramethyl-4-piperidyl)imino]-2-phenazinamine; |
| B4070 | N,5-*bis*(4-methylphenyl)-3,5-dihydro-3-[(4-piperidyl)imino]-2-phenazinamine; |
| B4103 | N,5-*bis*(4-trifluoromethylphenyl)-3,5-dihydro-3[(2',2',6',6'-tetramethyl-4-piperidyl)imino]-2-phenazinamine; |
| B4104 | N,5-*bis*(4-chlorophenyl)-8-chloro-3,5-dihydro-3-(cyclohexylimino)-2-phenazinamine; |
| B4123 | N,5-*bis*(3-chlorophenyl)-8-chloro-3,5-dihydro-3[(2,2',6',6'-tetramethyl-4-piperidyl)-imino]-2-phenazinamine; |
| B4126 | N,5-*bis*(3-trifluoromethyl-4-phenyl)-3,5-dihydro-3-[(2',2',6',6'-tetramethyl-4-piperidyl)-imino]-2-phenazinamine; |
| B4127 | N,5-*bis*(3-trifluoromethylphenyl)-8-chloro-3,5-dihydro-3-[(2',2',6',6'-tetramethyl-4-piperidyl)-imino]-2-phenazinamine; |
| B4154 | N,5-*bis*(3,4-di-chlorophenyl)-3,5-dihydro-3-[(3'-(N,N-diethylamino)-propylimino]-2-phenazinamine; |
| B4158 | N,5-*bis*(4-isopropylphenyl)-3,5-dihydro-3-[(2',2',6',6'-tetramethyl-4-piperidyl)-imino]-2-phenazinamine; |
| B4159 | N,5-*bis*(4-isopropylphenyl)-8-chloro-3,5-dihydro-3-[(2',2',6',6'-tetramethyl-4-piperidyl)-imino]-2-phenazinamine; |
| B4163 | N,5-*bis*[(3-trifluoromethyl)-4-chlorophenyl]-3,5-dihydro-3-[(2',2',6',6'-tetramethylpiperidyl)-imino]-2-phenazinamie. |
| B4166 | N,5-*bis*(phenyl)-3,5-dihydro-3-(cyclooctylimino)-2-phenazinamine; |
| B4169 | N,5-*bis*(3,4,5-trichlorophenyl)-3,5-dihydro-3[(2',2',6',6'-tetramethyl-4-piperidyl)-imino]-2-phenazinamine; |
| B4170 | N,5-*bis*(phenyl)-3,5-dihydro-3-(cyclopropylimino)-2-phenazinamine; |
| B4171 | N,5-*bis*(phenyl)-3,5-dihydro-3-(cyclododecylimino)-2-phenazinamine; |
| B4172 | N,5-*bis*(phenyl)-3,5-dihydro-3-(cyclobutylimino)-2-phenazinamine; |
| B4173 | N,5-*bis*(phenyl)-3,5-dihydro-3-[4'-(N-benzylpiperidyl)-imino]-2-phenazinamine; |
| B4174 | N,5-*bis*(4-methoxyphenyl)-3,5-dihydro-3-[(2',2',6',6'-tetramethyl-4-piperidyl)-imino]-2-phenazinamine; |
| B4175 | N,5-*bis*(3,4-di-chlorophenyl)-3,5-dihydro-3-(cyclohexylimino)-2-phenazinamine; |

The remaining compounds may be named in a similar manner.

Treatment of human or animal cells with a compound of the above general formula (I) results in the reduction of resistance to a wide variety of naturally anti-cancer drugs such as Vinca alkaloids, epipodophyllotoxenes, actinomycin D, anthracyclines, mitocycin C, mitoxantrone, taxol, and the like.

Without being bound by theory, the possible reasons for the surprising activity of the riminophenazines used in the invention is that a relationship may exist between riminophenazine mediated enhancement of PLA₂ activity and the inhibition of ATPase of P-glycoprotein, or the inhibition of P-glycoprotein activity may occur as a secondary consequence of the depletion of cellular ATP following prolonged inhibition of Na⁺, K⁺, ATPase activity. Thus, a reversal of multi-drug resistance may occur, primarily via activation of phospholipaseA₂ and consequent lysophospholipid-mediated inhibition of the ATPase activity of P-glycoprotein. Alternatively, inhibition of P-glycoprotein activity would be expected to occur as a secondary consequence of the depletion of cellular ATP following prolonged inhibition of Na⁺, K⁺-ATPase activity. Both of these mechanisms may be operative.

The riminophenazines of the above formula (I), contain an imino group. They are considerably less toxic than cyclosporin A and possess a potent resistant modifying activity in a multi-drug-resistant lung carcinoma cell line when administered *in vitro.* We have found that they inactivate the drug pump activity in tumour cell lines with acquired multi-drug resistance. The potency of compounds of the above general formula, as inhibitors of multi-drug resistance, was assessed by sensitization of a P-glycoprotein positive cell line to drugs associated with this form of resistance. A human small cell lung cancer cell line H 69/P as well as a multi-drug resistance (MDR) sub-line H69/LX4 were used. These cell lines were obtained from the Medical Research Council Clinical Oncology and Radiotherapeutics Unit, Hills Road, Cambridge, England. The H69/LX4 line was selected *in vitro* by progressive exposure to increasing concentrations of doxorubicin in order to increase P-glycoprotein expression.

A third cell line (562/MMB) used was a MDR leukemia cell line selected in vitro by progressive exposure to increasing concentrations of vinblastine in order to increase P-glycoprotein expression. This cell line was developed in the Department of Immunology, University of Pretoria from K562 (ATTC CCL 243) supplied by Highveld Biological (Pty) Ltd, Sandton. This cell line expresses high levels of P-glycoprotein as determined by flowcytometry using the monoclonal antibody (MRK 16) against this molecule.

The multi-drug resistance was increased substantially when using chemotherapeutic drugs such as doxorubicin, daunorubicin, etoposide and mitomycin C which are known to be associated with MDR. However, no increase in sensitivity was observed to any of the drugs tested in the sensitive parent cell line.

In addition to being relatively non-toxic, the compounds of the above general formula (I) are non-carcinogenic and non-myelosuppressive. They possess direct antineoplastic activity as well as multi-drug resistance modifying potential.

The compositions may be in any suitable form, e.g. a tablet, capsule, solution, sterile solution, or the like. They may contain any suitable known carrier or diluent. They may be introduced orally, intravenously, transdermally, or in any other suitable manner.

### DESCRIPTION OF THE DRAWINGS

Figure 1 is a chart showing the preparation of the riminophenazines of formula (I);
Figure 2 is a graph of cell viability against vinblastine concentration for compounds. B663, B669 and cyclosporin, as explained in Example 1 below;
Figure 3 is a graph of uptake of vinblastine against concentration of B663, B669 and cyclosporin as explained in Example 2 below;
Figure 4, 5, 6 and 7 are graphs of % inhibition against concentration, for the compounds B663, B669, B3962, B4154, B4176, B4100, B4090, B4070, B4174, B4169, B4103, B4126, B4158, B4159, B4163, B4127, B4123, B3786, B4019 and B4177 as explained in Example 3 below using the cell line H69/LX4, and
Figure 8 is a graph of % inhibition against concentration for the compounds B4128 and B4121, as explained in Example 4 below, using the cell like K562/MMB.

### DESCRIPTION OF EXAMPLES

The invention is illustrated in non-limiting manner by reference to the following Examples.

### Example A

In the Examples 1 to 3 given below, the potency of the riminophenazines, as well as cyclosporin A (CsA) was examined. A further test using no MDR inhibitor was used as a standard. The potency was assessed by sensitization of a P-glycoprotein positive cell line to doxorubicin, vinblastine, daunorubicin, mitomycin C, methotrexate and cyclophosphamide.

The cell lines used were human small cell lung cancer cell line HP69/P and a multi-drug resistant (MDR) sub-line H69/LX4, both of which were supplied by the MRC Clinical Oncology and Radiotherapeutics Unit, Hills Road, Cambridge, England. The maintenance of the cell lines and drug response assays was done as described by Twentyman *et al* in British Journal of Cancer Volume 65 pages 335-340. The H69/LX4 line was originally described by Twentyman *et al* in British *Journal* of Cancer Volume 53, pages 529-637 (1986). The drug resistance modifiers were added at concentrations which possess minimum cytotoxic activity.

### Example 1

In this Example, the effects were examined of a 7-day exposure of cyclosporin A (CsA) 5µg/mℓ, clofazimine at 1 µg/mℓ and B669 (the riminophenazine defined in Table 1 above) at 0,5µg/mℓ on the sensitivity of the cell line (H69/P) and the resistance cell line (H69/LX4) to the chemotherapeutic agents set out below, using the MTT assay. In this table, the IC50 (µg/mℓ)* of chemotherapeutic agents values are expressed as the mean drug concentration (µg/mℓ) causing 50% cell killing in 2 - 4 experiments. The data in parenthesis represents fold sensitivity compared with the chemotherapeutic agent without the MDR inhibitor.

The cells were seeded at 1 x 10⁴ cells per well in 96 well microlitre plates in a volume of 200 µℓ of RPMI 1640 medium containing 10% fetal calf serum and incubated with vinblastine (3,2-100 µg/mℓ). The amounts of MDR modulators used were 5 µg/mℓ of CsA, 1 µg/mℓ of clofazimine (B663) or 0,5 µg/mℓ of B669.

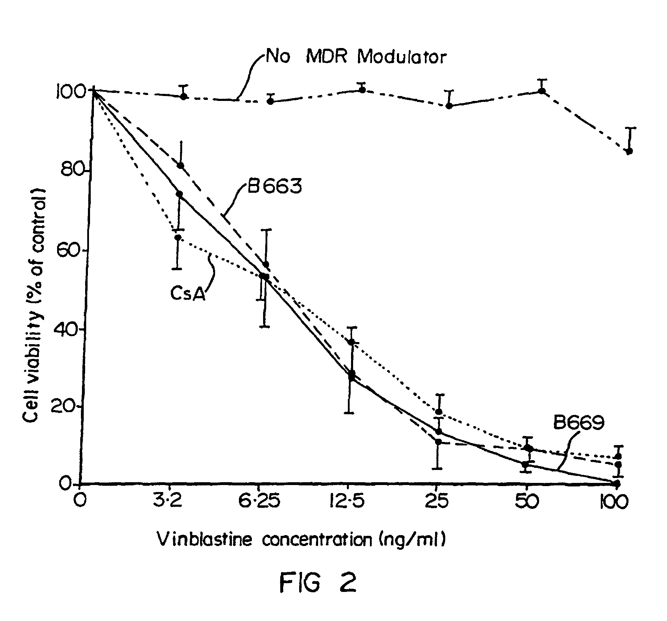

After incubation at 37°C for 7 days 20 µℓ MTT (ie 3-[4,5-dimethylthiazol-2-yl]-2,5-diphenyl-tetrazolium bromide) at 5 mg/mℓ was added to each well and the plates incubated for a further 4 hours. The cells were washed with phosphate buffered saline and the intracellular formazan crystals solubilized with dimethylsulphoxide, and the absorbence measured spectrophotometrically at a test wavelength of 540 nm and a reference wavelength of 620 nm. The mean percentage inhibition ± SEM of four experiments using the relevant controls was taken. The results obtained as shown in Table III below, are illustrated graphically in Figure 2 of the accompanying drawings.

**TABLE III**

| Effects of a 7 day Exposure to CsA (5 µg/ml), Clofazimine (1 µg/ml) and B669 (0.5 µg/ml) on the Sensitivity of Parent (H69/P) and Resistant (H69/LX4) Cells to Various Chemotherapeutic Agents Using the MTT Assay. | | | | |
|---|---|---|---|---|
| CHEMOTHERAPEUTIC | | IC50 (µg/ml)* | | |
| AGENTS | | MDR INHIBITOR | | |
| | None | CsA | Clofazimine (B663) | B669 |
| H69/P | | | | |
| Doxorubicin | 0.003 | 0.003 (1.0) | 0.004 (0.8) | 0.003 (1.0) |
| Vinblastine | 0.001 | 0.0007 (1.4) | 0.0008 (1.3) | 0.0009 (1.1) |
| Etoposide | 0.0007 | 0.00009(0.8) | 0.00011(0.6) | 0.00006(1.2) |
| Daunorubicin | 0.002 | 0.002 (1.0) | 0.002 (1.0) | 0.0016 (1.2) |
| Mitomycin C | 0.007 | 0.009 (0.8) | 0.01 (0.7) | 0.01 (0.7) |
| Methotrexate | 3.5 | 2.4 (1.5) | 3.7 (0.9) | 3.4 (1.0) |
| Cyclophospha -mide | 1.8 | 1.6 (1.1) | 1.9 (0.9) | 1.8 (1.0) |

| H69/LX4 | | | | |
|---|---|---|---|---|
| Doxorubicin | 0.11 | 0.01 (11) | 0.01 (11) | 0.016 (6.9) |
| Vinblastine | 0.120 | 0.008 (13.7) | 0.008 (13.7) | 0.008 (13.7) |
| Etoposide | 0.013 | 0.00008(16.3) | 0.0026(5.0) | 0.00009(14.4) |
| Daunorubicin | 0.036 | 0.004 (9) | 0.009 (4) | 0.015 (2.4) |
| Mitomycin C | 0.1 | 0.08 (1.3) | 0.06 (1.7) | 0.04 (2.5) |
| Methotrexate | 3.2 | 4.6 (0.7) | 4.4 (0.7) | 3.5 (0.9) |
| Cyclophosphamide | 1.6 | 1.7 (0.9) | 1.6 (1.0) | 1.4 (1.1) |

| | | | | |
|---|---|---|---|---|
| * Clofazimine (at 1 µg/ml) and B669 (at only 0,5 µg/ml) compared favourably with CsA (at the much higher dosage of 5 µg/ml) in reversing the MDR of Vinblastine. Similar results were obtained with the other MDR - related drugs Doxorubicin, Daunorubicin and Mitomycin C. | | | | |

As can be seen, clofazimine at (1 µg/mℓ) and B669 (at 0.5 µg/mℓ) compared favourably with cyclosporin A (at the much higher dose of 5 µg/mℓ) in reversing multi-drug resistance by as much as a 13 fold increase of the MDR cell line (H69/LX4) to vinblastine as well as giving similar results with the other MDR-related chemotherapeutic drugs, namely Doxorubicin, Etoposide, Daunorubicin and Mitomycin C, but not with drugs which are not associated with MDR, ie methotrexate and cyclophosphamide.

### Example 2

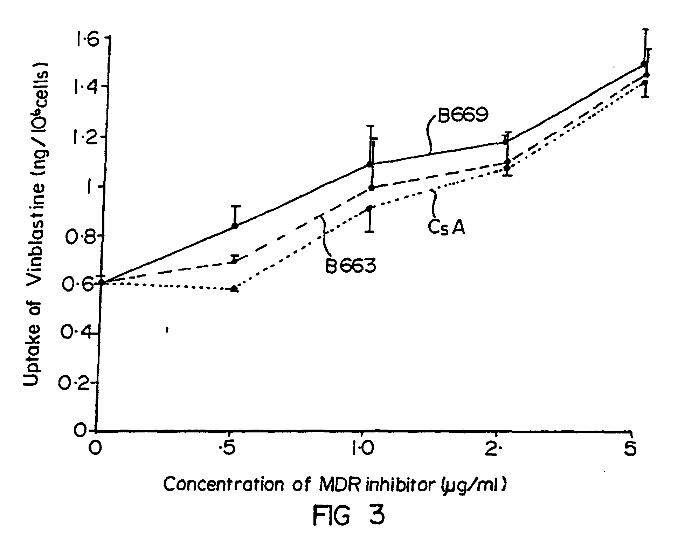

The effect of riminophenazines on the accumulation of [¹⁴C]-vinblastine was investigated. For these experiments, both of the above cell lines (H69/LX4 and H69/P) were prepared as described by Coley *et al* (Biochem. Pharmacal 38, 4467-4475 (1989)). After a 1 hour pretreatment with the MDR reversal agents, cells (1 x 10⁶/mℓ) were exposed to 250 µg/mℓ [¹⁴C]-vinblastine (1 µCi specific activity) for 30 minutes. Clofazimine, B669 and cyclosporin A caused significant enhancement of vinblastine accumulation in the MDR cell line (H69/LX4). The results are illustrated in Figure 3 of the accompanying drawings. These effects were dose related and observed at concentrations of 0.5 µg/mℓ and upwards with the riminophenazine, and at concentrations of 1 µg/mℓ and greater with cyclosporin A. However, the MDR inhibitors had no effect on vinblastine accumulation in the sensitive parent cell line (H69/P). Total vinblastine uptake by H69/P cells was 3.01 ±0.40 µg/10⁶ cells without MDR modifying agents and 2.95 ± 0,10, 2,69 ±0.23 and 2.56 ± 0,07 µg/10⁶ cells with 5 µg/mℓ of cyclosporin A, clofazimine and B669 respectively.

### Example 3

The effects were examined of a 7-day exposure of selected compounds on the sensitivity of the cell line (H69/P) and the resistance cell line (H69/LX4) to the standard chemotherapeutic agents doxorubicin and vinblastine, using the MTT assay.

The cells were seeded at 1 x 10⁴ cells per well in 96 well microlitre plates in a volume of 200 µℓ of RPMI 1640 medium containing 10% fetal calf serum and incubated with doxorubicin (12,5 ng/ml) or vinblastine (25 ng/ml). The amounts of MDR modulators used were 0,03; 0,06; 0,125; 0,25; 0,5; 1,0 and 2,0 µg/ml.

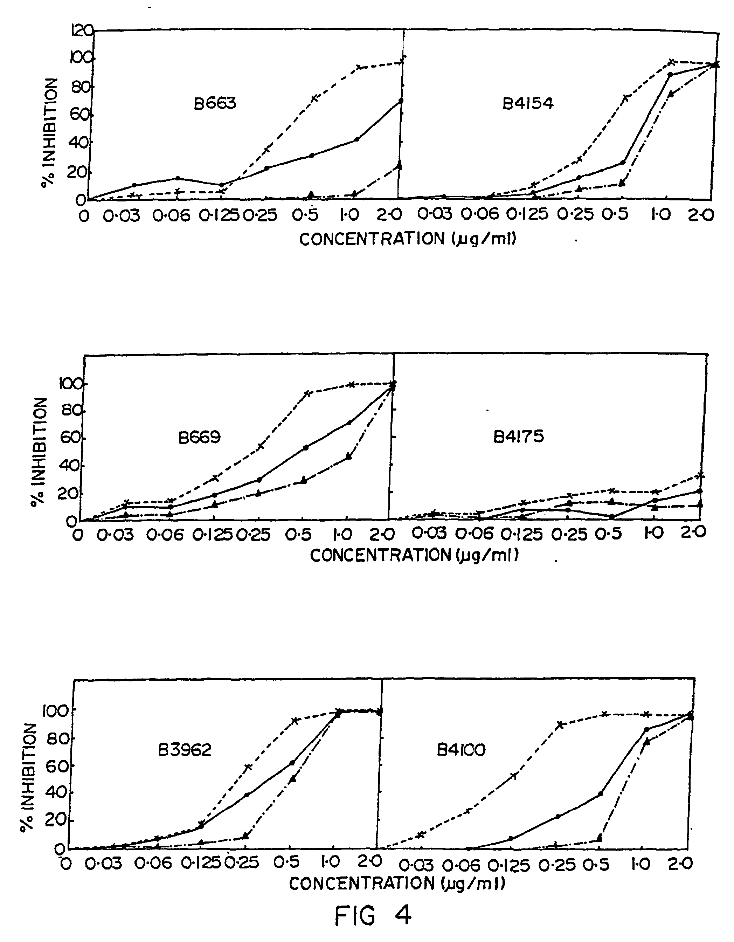

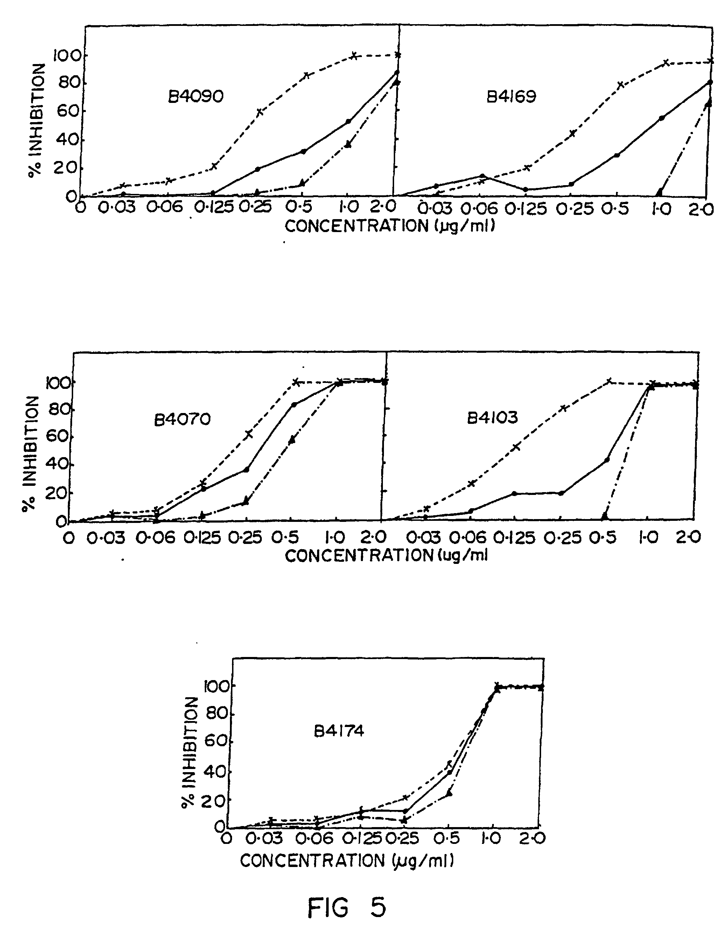

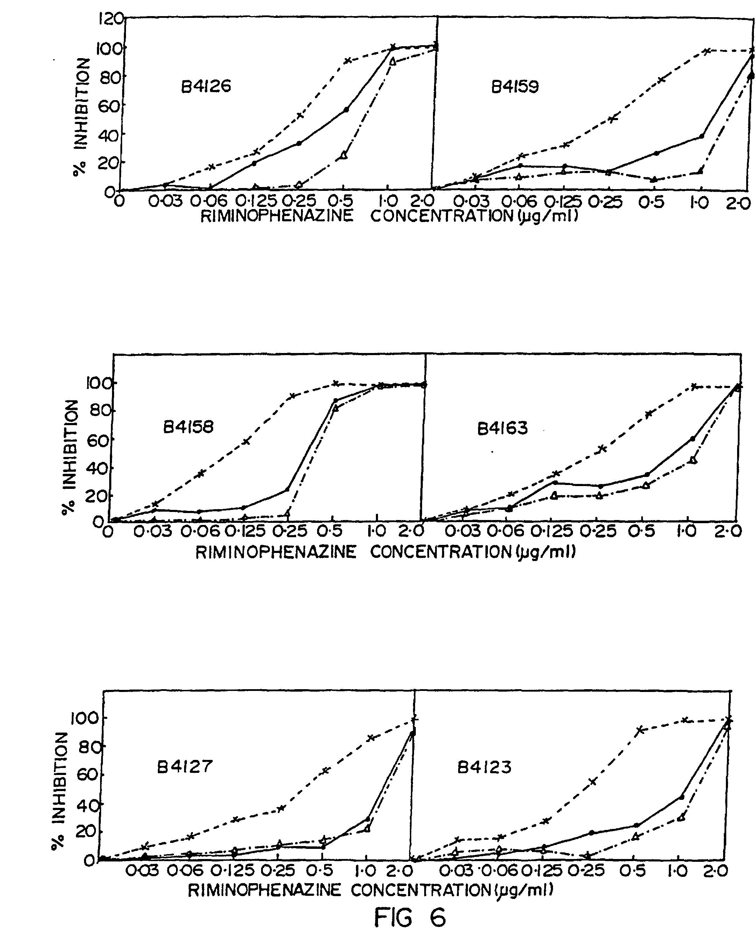

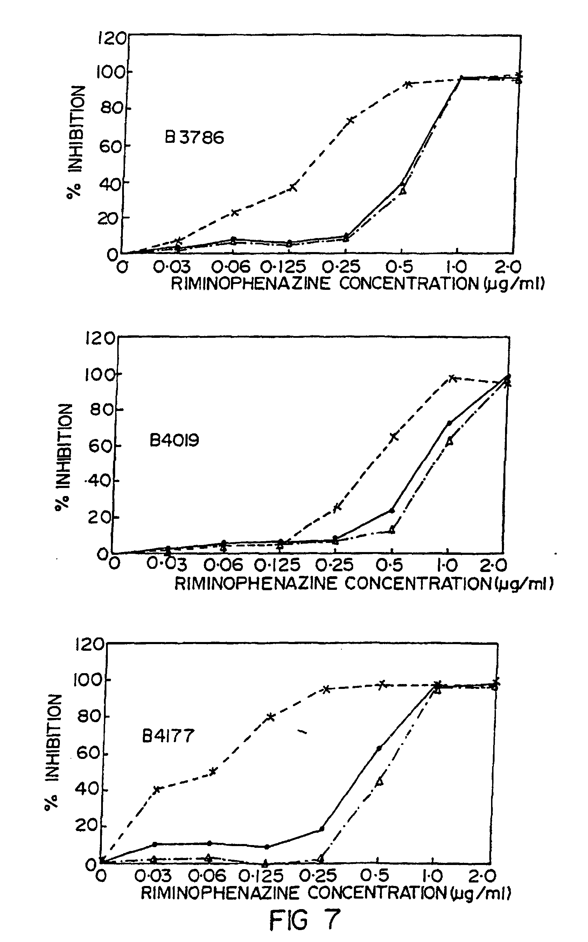

After incubation at 37°C for 7 days, 20 µℓ MTT (ie 3-[4,5-dimethylthiazol-2-yl]-2,5-diphenyl-tetrazolium bromide) at 5 mg/ml was added to each well and the plates incubated for a further 4 hours. The cells were washed with phosphate buffered saline and the intracellular formazan crystals solubilized with dimethylsulphoxide, and the absorbence measured spectrophotometrically at a test wavelength of 540 nm and a reference wavelength of 620 nm. The mean percentage inhibition ± SEM of four experiments using the relevant controls was taken. The results obtained, are illustrated graphically in Figures 4 to 7 of the accompanying drawings.

The drawings in Figures 4, 5, 6 and 7 and are graphs for the % inhibition of the compound against the riminophenazine concentration (in µg/mg). The mixed broken and solid line (marked with triangles) is for the compound alone, the solid line (.-.) shows the result when 12,5 ng/ml of doxorubicin are present, and the broken line (x-x) shows the results when 25 ng/ml of vinblastine are present. The concentrations of doxorubicin and vinblastine (present with the riminophenazine) possessed minimal cytotoxic activity (less than 10 %). Of particular interest is the difference between direct cytotoxicity, (i.e. the effects of the compounds alone) and the MDR-reversal properties, (i.e. the effects of the compounds in the presence of vinblastine and dexorubucin).

The compounds tested and whose results are shown graphically were B663, B669, B3962, B4070, B4100, B4103, B4123, B4126, B4127, B4154, B4158, B4159, B4163, B4169, B4174 and B4176.

Very good MDR reversal properties, (i.e. multi-drug resistance) were shown by this test were for B4169, B4158 and B4103. B4169 appears to be of particular potential in view of its very small cytotoxic activity (only cytotoxic at concentrations above 12,5 µg/ml). Also important is the large differences in % inhibition at a number of concentrations between the riminophenazines tested and vinblastine.

### Example 4

The effects were examined of a 7-day exposure of selected compounds on the sensitivity of the cell line (K562/MMB) to the standard chemotherapeutic agents doxorubicin and vinblastine, using the MTT assay.

The cells were seeded at 1 x 10⁴ cells per well in 96 well microlitre plates in a volume of 200 µl of RPMI 1640 medium containing 10 % fetal calf serum and incubated with doxorubicin (12.5 ng/ml) or vinblastine (3 ng/ml). The amounts of MDR modulators used were 0.03, 0.06, 0.125, 0.5, 1.0 and 2.0 µg/ml.

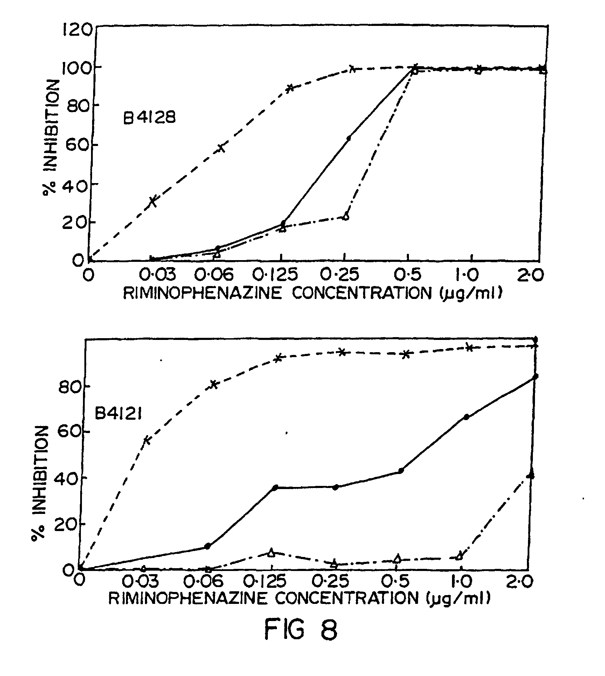

After incubation at 37 °C for 7 days, 20 µl MTT (i.e. 3-[4,5-dimethylthiazol-2-yl]2,5-diphenyl-tetrazolium bromide) at 5 mg/ml was added to each well and the plates incubated for a further 4 hours. The cells were washed with phosphate buffered saline and the intracellular formazan crystals solubilized with dimethylsulphoxide, and the absorbency measured spectrophotometrically at a test wavelength of 540 nm and a reference wavelength of 620 nm. The results obtained are shown in Table IV and Figure 8. In this table the IC 50 (µg/ml)* of riminophenazines are expressed as the mean drug concentration (µg/ml) causing 50% cell killing in 2 experiments. The concentrations of doxorubicin and vinblastine (present with the riminophenazine) possessed minimal cytotoxic activity (less than 10%).

The compounds tested and whose results are shown in the table are B663, B669, B4100, B4158, B4169, B4103, B4126, B4159, B4163, B4127, B4123, B3962, B4090, B4070, B4174, B4154, B4157, B4121 and B4128.

Very good MDR reversal properties (at concentrations less than 0.05 µg/ml causing 50 % cell killing in the presence of 3 ng/ml vinblastine) shown by this test were for B663, B669, B4163, B4123, B4090 and B4121. B4121 and B4169 appear to be of particular potential in view of their very small cytotoxic activity (only causing 50 % cell killing per se at 2.250 and 2.861 µg/ml respectively) whereas B4121 also possesses good MDR reversal properties (causing 50 % cell killing in the presence of 3 ng/ml vinblastine at 0.023 µg/ml).

**TABLE IV**

| Effects of a 7 day exposure to different concentration of various riminophenazine compounds on the sensitivity of a doxorubicin resistant leukaemia cell line (K562/MMB) to doxorubicin (12 ng/ml) or vinblastine (3 ng/ml) using the MTT assay. | | | |
|---|---|---|---|
| Riminophenazine agents | | IC 50 (µg/ml)* | |
| | None | Doxorubicin | Vinblastine |
| B663 | 0.225 | 0.135 | 0.038 |
| B669 | 0.139 | 0.069 | 0.023 |
| B4100 | 0.335 | 0.278 | 0.095 |
| B4158 | 0.272 | 0.155 | 0.058 |
| B4169 | 2.861 | 0.969 | 0.116 |
| B4103 | 0.351 | 0.269 | 0.051 |
| B4126 | 0.316 | 0.246 | 0.071 |
| B4159 | 0.912 | 0.514 | 0.130 |
| B4163 | 0.340 | 0.227 | 0.032 |
| B4127 | 0.425 | 0.168 | 0.070 |
| B4123 | 0.333 | 0.181 | 0.049 |
| B3962 | 0.285 | 0.159 | 0.070 |
| B4090 | 0.197 | 0.139 | 0.027 |
| B4070 | 0.159 | 0.193 | 0.054 |
| B4174 | 0.316 | 0.210 | 0.178 |
| B4154 | 0.353 | 0.296 | 0.118 |
| B4157 | >2.0 | >2.0 | >2.0 |
| B4121 | 2.250 | 0.722 | 0.023 |
| B4128 | 0.383 | 0.215 | 0.059 |

### Example 5

Some compositions of the invention are made up as follows:

| CAPSULES | mg/capsule |
|---|---|
| Riminophenazine | 100 - 2000 mg |
| Diluent/Disintegrant | 5 - 200 mg |
| Glidants | 0 - 15 mg |
| Disintegrants | 0 - 20 mg |

| TABLETS | mg/tablet |
|---|---|
| Riminophenazine | 100 - 2000 mg |
| Diluent | 5 - 200 mg |
| Disintegrant | 2 - 50 mg |
| Binder | 5 - 100 mg |
| Lubricant | 2 - 20 mg |

| SYRUP | mg/10 ml |
|---|---|
| Riminophenazine | 100 - 2000 mg |
| Solvents, solubilisers, stabilisers | 5 - 500 mg |
| Colouring agents | 0,5 - 150 mg |
| Preservatives/Antioxidants | 1 - 150 mg |
| Flavours | 5 - 200 mg |

| INTRAVENOUS | |
|---|---|
| Riminophenazine | 100 - 2000 mg |
| Alkali/buffer, Isotonically agents | 5 - 100 µg |
| Stabilisers, solubilisers | 0 - 100 µg |

## Claims

1. The use of a riminophenazine in the manufacture of a medicament to treat a cancer patient who has built up, or could build up, resistance to a therapeutically active substance used in the treatment of cancer.

2. The use of the riminophenazine as claimed in Claim 1, wherein said riminophenazine is of the following general formula (I) - 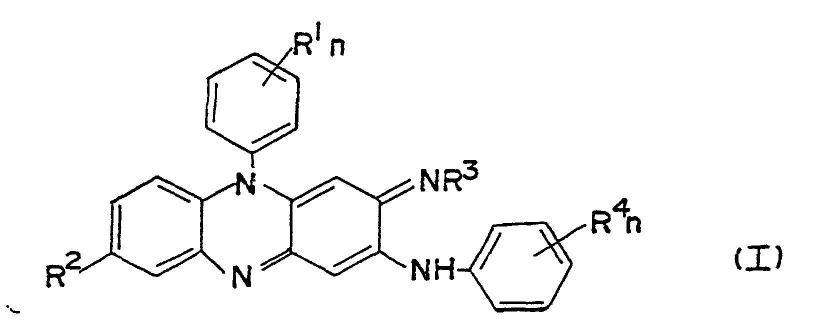 in which:
R¹ and R⁴ are the same and each is a halogen atom, or a (C₁ - C₄)- alkyl, (C₁-C₄)-alkoxy, (C₁-C₄)-fluoroalkoxy or haloalkyl radical,
R² is a hydrogen or halogen atom,
R₃ is a 4-tetramethylpiperidyl radical and n is 1,2 or 3 with the provisos that (a) when n is 2 or 3, (R¹)n and (R⁴)n are not 3,4-dichloro and that (b) when n is 1, substituents R¹ and R⁴ cannot represent (C₁-C₄)-alkyl or (C₁-C₄)-alkoxy radicals or a halogen in the 4-position.

3. The use of the riminiophenazine as claimed in Claim 1, wherein said riminophenazine is of the following general formula (I) - 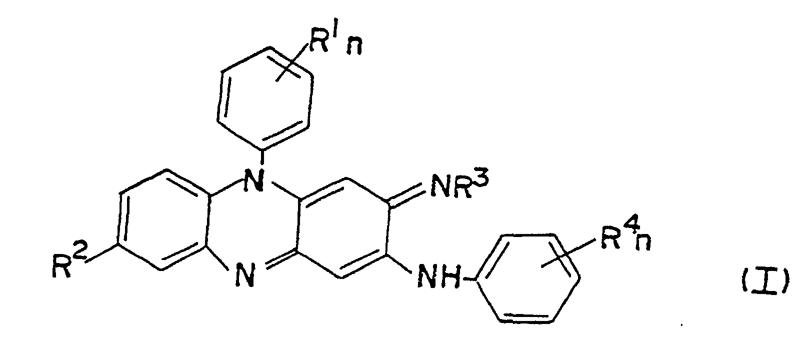 in which
R¹ and R⁴ are the same and each is a hydrogen atom, a halogen atom, or an alkyl radical,
R² is a hydrogen or halogen atom,
R³ is selected from hydrogen, alkyl, substituted alkyl, cycloalkyl, cycloalkylalkyl, or is a heterocyclic or heterocyclic-alkyl radical, and
n is 1, 2 or 3.

4. The use as claimed in claim 2, of a riminophenazine of general formula I, in which R¹ and R⁴ are the same and each is a trifluoromethyl radical, R² is a hydrogen or halogen atom, R³ is a 4-tetramethylpiperidyl n is 1, 2 or 3.

5. The use as claimed in Claim 2 or claim 4 wherein R³ is a 4-(2,2,6,6, - tetramethylpiperidyl) radical.

6. The use as claimed in Claim 2, wherein the haloalkyl radicals R¹ and R⁴ are trifluoromethyl radicals.

7. The use of a riminophenazine as claimed in claim 3, wherein R¹ and R⁴ are 4-chloro, R² is hydrogen, R³ is isopropyl and n is 1, i.e the compound clofazimine.

8. The use as claimed in claims 2 and 4 of a compound of formula (I), wherein n is 1, R¹ and R⁴ each is a trifluoromethyl radical in the 4-position, R² is hydrogen and R³ is a 4-(2,2,6,6,-tetramethylpiperidyl) radical.

9. The use as claimed in Claim 2 of a compound of formula (I), wherein n is 2, R¹ and R⁴ are identical and are chlorine atoms in the 2,4 -, 3,4 or 3,5 - positions, R² is hydrogen and R³ is a 4 - (2,2,6,6-tetramethylpiperidyl) radical.

10. The use as claimed in claim 2 of a compound according to formula (I), wherein n is 3, R¹ and R⁴ are chlorine atoms in the 3-,4- and 5-positions, R² is hydrogen and R³ is a 4-(2,2,6,6,-tetramethylpiperidyl) radical.

11. A compound of general formula (I) 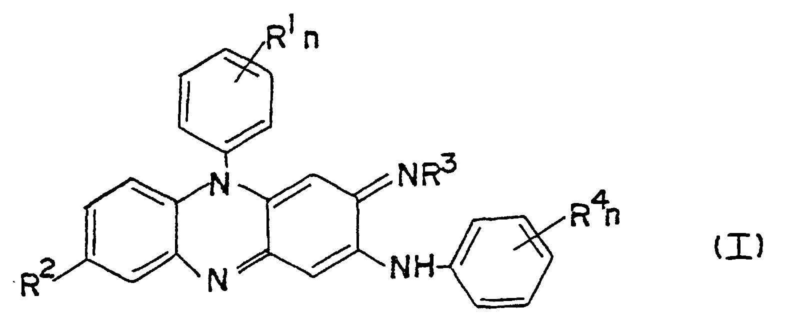 in which -
R¹ and R⁴ are the same and are selected from halogen, (C₁-C₄)-alkyl, (C₁-C₄)-alkoxy, (C₁-C₄)-fluoroalkoxy and trifluoromethyl radicals n is 1, 2 or 3, R³ is a 4-(2,2,6,6-tetramethylpiperidyl) radical and R² is selected from hydrogen and halogen with the proviso that (a) when n is 2 or 3, (R¹)n and (R⁴)n are not 3,4 - dichloro and that (b) when n = 1, substituents R¹ and R⁴ cannot represent (C₁-C₄)-alkyl or (C₁-C₄)-alkoxy radicals or a halogen in the 4-position.

12. A compound accordingly to Claim 11, wherein the phenyl rings containing (R¹)n and (R⁴)n are 3,5-dichlorophenyl rings, R² is hydrogen and R³ is a 4-(2,2,6,6,-tetramethylpiperidyl) radical, i.e. the compound N,5-*bis* (3,5-dichlorophenyl)-3,5-dihydro-3-[(2',2',6',6'-tetramethylpiperidyl)imino]-2-phenazinamine.

13. A compound according to claim 11, wherein the phenyl rings containing (R¹)n and (R⁴)n are 2,4-dichlorophenyl rings, R² is hydrogen and R³ is a 4-(2,2,6,6-tetramethylpiperidyl) radical, i.e. the compound N,5-bis(2,4 dichlorophenyl)-3,5-dihydro-3-[(2',2',6',6' - tetramethylpiperidyl)imino]-2-phenazinamine.

14. A compound according to claim 11, wherein R¹ and R⁴ are chlorine atoms in the 3-,4- and 5-positions, R² is hydrogen and R³ is a 4-(2,2,6,6-tetramethylpiperidyl). radical, i.e. the compound N,5-*bis*(3,4,5-trichlorophenyl)-3,5-dihydro-3-[(2',2',6',6' -tetramethylpiperidyl)imino]-2-phenazinamine.

15. A compound according to claim 11, wherein the phenyl rings containing (R¹)n and (R⁴)n are 3-trifluoromethyl-4-chlorophenyl rings, R²is hydrogen and R³ is a 4-(2,2,6,6-tetramethylpiperidyl) radical, i.e. the compound N,5-*bis*(3-trifluoromethyl-4-chlorophenyl)-3,5-dihydro-3-[(2',2'16',6'-tetramethylpiperidyl) imino]-2-phenazinamine.

16. A compound according to claim 11 and which is N,5-*bis*(4-trifluoromethylphenyl)-3,5-dihydro-3-[2',2',6,'6'-tetramethylpiperidyl)imino]-2-phenazinamine.

17. A compound according to claim 11 and which is N,5-*bis*(3-trifluoromethylphenyl)-3,5-dihydro-3[(2',2',6'-tetramethyl-4-piperidyl)imino]-2- phenazinamine.

18. A compound according to claim 11 and which is N,5-*bis*(4-trifluoromethoxyphenyl)-3,5-dihydro-3-[(2',2',6',6'-tetramethyl-4-piperidyl)imino]-2-phenazinamine.

19. A compound according to claim 11 and which is N,5-*bis*(3-trifluoromethylphenyl)-8-chloro-3,5-dihydro-3-[(2',2',6',6'-tetramethyl-4-piperidyl)imino]-2-phenazinamine.

20. A compound of the formula (I) 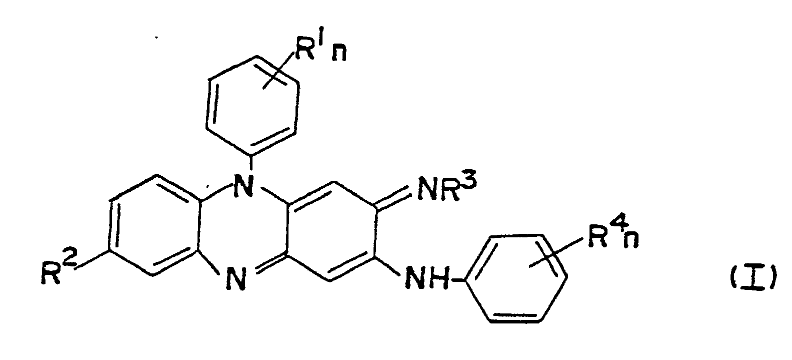 wherein the compound is selected from the group consisting of-
(a) compound B4123 i.e. N,5-*bis*(3-chlorophenyl)-8-chloro-3[(2',2',6',6'-tetramethyl-4-piperidyl)imino]-2-phenazinamine,
(b) compound B4158 i.e. N,5-*bis*(4-isopropylphenyl)-3,5-dihydro-3-[(2',2',6',6'-tetramethyl-4-piperidyl)imino]-2-phenzinamine,
(c) compound B4159 i.e. N,5-*bis*(4-isopropylphenyl)-8-chloro-3,5-dihydro-3-[(2',2',6',6'-tetramethyl-4-piperidyl)imino]-2-phenazinamine and
(d) compound B4174 i.e. N,5-*bis*(4-methoxyphenyl)-3,5-dihydro-3-[(2',2',6',6'-tetramethyl-4-piperidyl)imino]-2-phenazinamine.

21. A composition for the treatment of a cancer patient who has built up, or could build up, resistance to a therapeutically active substance used in the treatment of cancer, said composition containing a compound as claimed in any of claims 11 to 20 and a pharmaceutically acceptable carrier.

22. A composition as claimed in claim 21, wherein the composition also contains a known cancer treatment compound which is not a riminophenazine.

23. A method for the preparation of a compound of the general formula (I) 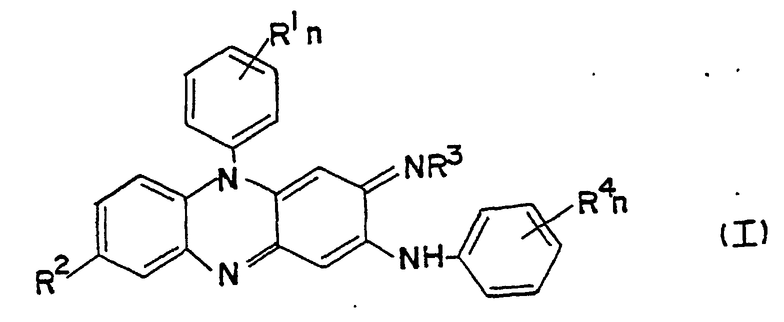 in which
- R¹,R²,R³,R⁴, and n have the meanings defined claim 11,
which comprises reacting a compound of formula (IV) - 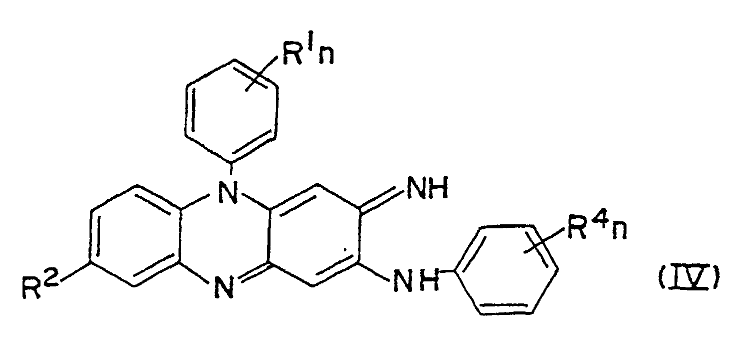 in which
with a compound of formula R³-NH₂.
in which R¹, R², R⁴ and n are as defined above.

## Patentansprüche

1. Die Verwendung eines Riminophenazins bei der Herstellung eines Medikaments zur Behandlung eines Krebspatienten, der gegenüber einer therapeutisch wirksamen Substanz, die bei der Krebsbehandlung verwendet wird, resistent ist oder werden könnte.

2. Verwendung des Riminophenazins gemäß Anspruch 1, wobei das Riminophenazin aus der folgenden allgemeinen Formel (I) besteht: 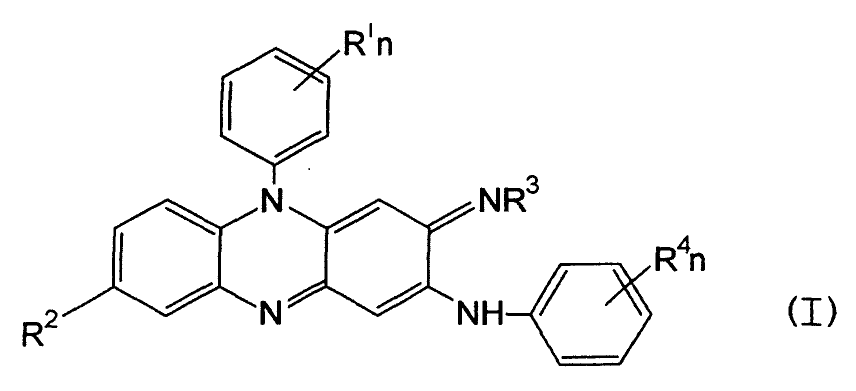 wobei:
R¹ und R⁴ gleich sind und jedes ein Halogenatom oder ein (C₁ - C₄)-alkyl-, (C₁ - C₄)-alkoxy-, (C₁ - C₄)-fluoralkoxy- oder Halogenalkylradikal ist,
R² ein Wasserstoff- oder Halogenatom ist,
R³ ein 4-Tetramethylpiperidylradikal und n 1, 2 oder 3 ist, unter der Bedingung, dass (a) wenn n 2 oder 3 ist, (R¹)n und (R⁴)n kein 3,4-Dichlor sind, und dass (b) wenn n 1 ist, die Substituenten R¹ und R⁴ keine (C₁ - C₄)-alkyl- oder (C₁ - C₄)-alkoxyradikale oder ein Halogen in der 4-Position repräsentieren können.

3. Verwendung des Riminophenazins gemäß Anspruch 1, wobei das Riminophenazin aus der folgenden allgemeinen Formel (I) besteht: 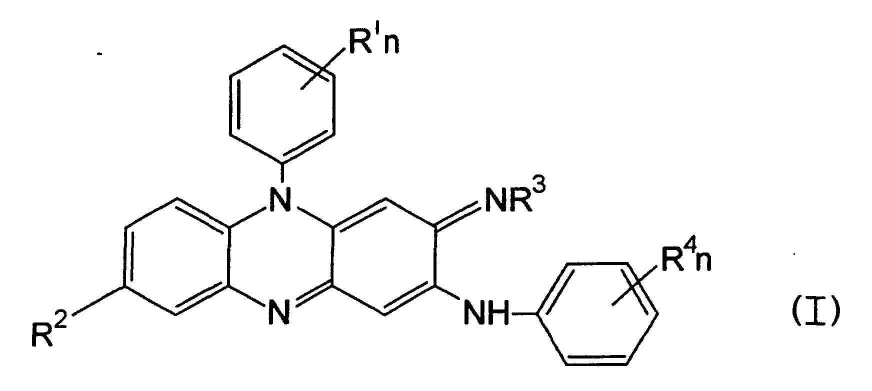 wobei
R¹ und R⁴ gleich sind und jedes ein Wasserstoffatom, ein Halogenatom oder ein Alkylradikal ist,
R² ein Wasserstoff- oder Halogenatom ist,
R³ aus Wasserstoff, Alkyl, substituiertem Alkyl, Cycloalkyl, Cycloalkyl-alkyl ausgewählt wird oder ein heterocyclisches Radikal oder heterocyclisches Alkyradikal ist, und n 1, 2 oder 3 ist.

4. Verwendung gemäß Anspruch 2 eines Riminophenazins von der allgemeinen Formel I, wobei R¹ und R⁴ gleich sind und jedes ein Trifluormethylradikal ist, R² ein Wasserstoff- oder Halogenatom ist, R³ ein 4-Tetramethylpiperidyl ist, n 1, 2 oder 3 ist.

5. Verwendung gemäß Anspruch 2 oder Anspruch 4, wobei R³ ein 4-(2,2,6,6-Tetramethylpiperidyl)radikal ist.

6. Verwendung gemäß Anspruch 2, wobei die Halogenalkylradikale R¹ und R⁴ Trifluormethylradikale sind.

7. Verwendung eines Riminophenazins gemäß Anspruch 3, wobei R¹ und R⁴ 4-Chlor sind, R² Wasserstoff ist, R³ Isopropyl ist und n 1, d.h. die Verbindung Clofazimin ist.

8. Verwendung gemäß Ansprüchen 2 und 4 einer Verbindung der Formel (I), wobei n 1 ist, R¹ und R⁴ jedes ein Trifluormethylradikal in der 4-Position sind, R² Wasserstoff ist und R³ ein 4-(2,2,6,6-Tetramethylpiperidyl)radikal ist.

9. Verwendung gemäß Anspruch 2 einer Verbindung der Formel (I), wobei n 2 ist, R¹ und R⁴ identisch sind und Chloratome in den 2,4-, 3,4- oder 3,5-Positionen sind, R² Wasserstoff ist und R³ ein 4-(2,2,6,6-Tetramethylpiperidyl)radikal ist.

10. Verwendung gemäß Anspruch 2 einer Verbindung nach Formel (I), wobei n 3 ist, R¹ und R⁴ Chloratome in den 3-, 4- und 5-Positionen sind, R² Wasserstoff ist und R³ ein 4-(2,2,6,6-Tetramethylpiperidyl)radikal ist.

11. Eine Verbindung der allgemeinen Formel (I) 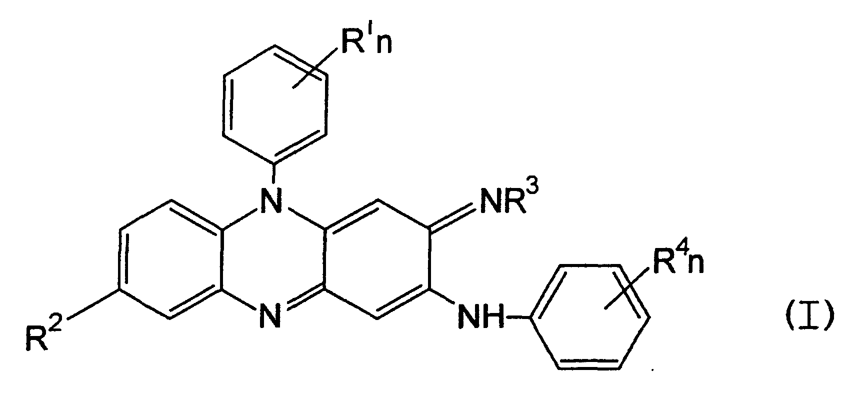 wobei:
R¹ und R⁴ gleich sind und aus Halogen, (C₁ - C₄)-alkyl-, (C₁ - C₄)-alkoxy-, (C₁ - C₄)-fluoralkoxy- und Trifluormethylradikalen ausgewählt sind, n 1, 2 oder 3 ist, R³ ein 4-(2,2,6,6-Tetramethylpiperidyl)radikal ist und R² aus Wasserstoff und Halogen ausgewählt wird, unter der Bedingung, dass (a) wenn n 2 oder 3 ist, (R¹)n und (R⁴)n kein 3,4-Dichlor sind, und dass (b) wenn n = 1 ist, die Substituenten R¹ und R⁴ keine (C₁ - C₄)-alkyl- oder (C₁ - C₄)-alkoxyradikale oder ein Halogen in der 4-Position repräsentieren können.

12. Verbindung gemäß Anspruch 11, wobei die (R¹)n und (R⁴)n enthaltenden Phenylringe 3,5-Dichlorphenylringe sind, R² Wasserstoff ist und R³ ein 4-(2,2,6,6-Tetramethylpiperidyl)radikal ist, d.h. die Verbindung N,5-*bis*(3,5-Dichlorphenyl)-3,5-dihydro-3-[(2',2',6',6'-Tetramethylpiperidyl)-imino]-2-phenazinamin ist.

13. Verbindung gemäß Anspruch 11, wobei die (R¹)n und (R⁴)n enthaltenden Phenylringe 2,4-Dichlorphenylringe sind, R² Wasserstoff und R³ ein 4-(2,2,6,6-Tetramethylpiperidyl)radikal ist, d.h. die Verbindung N,5-bis(2,4-Dichlorphenyl)-3,5-Dihydro-3-[(2',2',6',6'-Tetramethylpiperidyl)-imino]-2-Phenazinamin ist.

14. Verbindung gemäß Anspruch 11, wobei R¹ und R⁴ Chloratome in den 3-, 4- und 5-Positionen sind, R² Wasserstoff ist und R³ ein 4-(2,2,6,6-Tetramethylpiperidyl)radikal ist, d.h. die Verbindung N,5-*bis*(3,4,5-Trichlorphenyl)-3,5-Dihydro-3-[(2',2',6',6'-Tetramethylpiperidyl)-imino]-2-Phenazinamin ist.

15. Verbindung gemäß Anspruch 11, wobei die (R¹)n und (R⁴)n enthaltenden Phenylringe 3-Trifluormethyl-4-Chlorphenylringe sind, R² Wasserstoff ist und R³ ein 4-(2,2,6,6-Tetramethylpiperidyl)radikal ist, d.h. die Verbindung N,5-*bis*(3-Trifluormethyl-4-Chlorphenyl)-3,5-Dihydro-3-[(2',2',6',6'-Tetramethylpiperidyl)-imino]-2-Phenazinamin ist.

16. Verbindung gemäß Anspruch 11 und die N,5-*bis*(4-Trifluormethylphenyl)-3,5-Dihydro-3-[(2',2',6',6'-Tetramethylpiperidyl)-imino]-2-Phenazinamin ist.

17. Verbindung gemäß Anspruch 11 und die N,5-*bis*(3-Trifluormethylphenyl)-3,5-Dihydro-3[(2',2',6'-Tetramethyl-4-Piperidyl)-imino]-2-Phenazinamin ist.

18. Verbindung gemäß Anspruch 11 und die N,5-*bis*(4-Trifluormethoxyphenyl)-3,5-Dihydro-3-[(2',2',6',6'-Tetramethyl-4-Piperidyl)-imino]-2-Phenazinamin ist.

19. Verbindung gemäß Anspruch 11 und die N,5-*bis*(3-Trifluormethylphenyl)-8-Chlor-3,5-Dihydro-3-[(2',2',6',6'-Tetramethyl-4-Piperidyl)-imino]-2-Phenazinamin ist.

20. Verbindung der Formel (I) 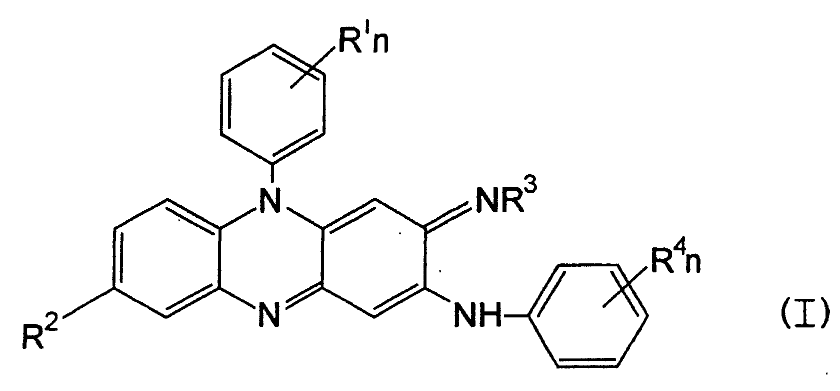 wobei die Verbindung aus der Gruppe ausgewählt wird, die aus folgendem besteht:
(a) Verbindung B4123, d.h. N,5-*bis*(3-Chlorphenyl)-8-Chlor-3[(2',2',6',6'-Tetramethyl-4-Piperidyl)-imino]-2-Phenazinamin,
(b) Verbindung B4158, d.h. N,5-*bis*(4-Isopropylphenyl)-3,5-Dihydro-3-[(2',2',6',6'-Tetramethyl-4-Piperidyl)-imino]-2-Phenazinamin,
(c) Verbindung B4159, d.h. N,5-*bis*(4-lsopropylphenyl)-8-Chlor-3,5-Dihydro-3-[(2',2',6',6'-Tetramethyl-4-Piperidyl)-imino]-2-Phenazinamin und
(d) Verbindung B4174, d.h. N,5-*bis*(4-Methoxyphenyl)-3,5-Dihydro-3-[(2',2',6',6'-Tetramethyl-4-Piperidyl)-imino]-2-Phenazinamin.

21. Eine Zusammensetzung zur Behandlung eines Krebspatienten, der gegenüber einer therapeutisch wirksamen Substanz, die bei der Behandlung von Krebs verwendet wird, resistent ist oder werden könnte, wobei die Zusammensetzung eine Verbindung gemäß einem der Ansprüche 11 bis 20 und einen pharmazeutisch akzeptablen Träger enthält.

22. Zusammensetzung gemäß Anspruch 21, wobei die Zusammensetzung auch eine bekannte Krebsbehandlungsverbindung, die kein Riminophenazin ist, enthält.

23. Ein Verfahren zur Herstellung einer Verbindung der allgemeinen Formel (I) 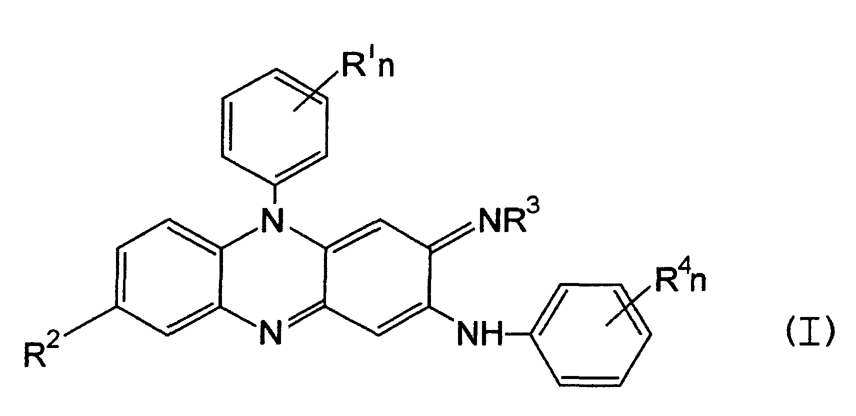 wobei:
R¹, R², R³, R⁴ und n die in Anspruch 11 definierten Bedeutungen haben,
wobei dieses das zur-Reaktion-bringen einer Verbindung folgender Formel (IV) umfasst: 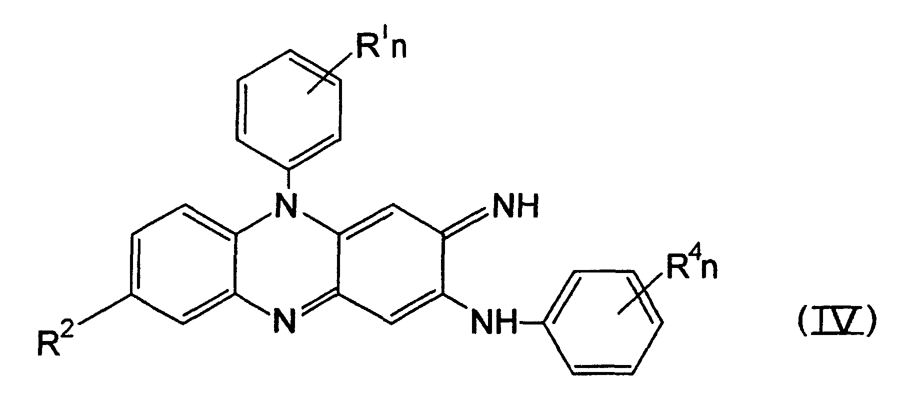 wobei
mit einer Verbindung der Formel R³-NH₂
wobei R¹, R², R⁴ und n wie oben definiert sind.

## Revendications

1. L'utilisation d'une riminophénazine dans la fabrication d'un médicament pour traiter un patient cancéreux qui a développé, ou pourrait développer, une résistance à une substance thérapeutiquement active utilisée dans le traitement du cancer.

2. L'utilisation de la riminophénazine telle que revendiquée dans la revendication 1, dans laquelle ladite riminophénazine est de la formule générale suivante (I) 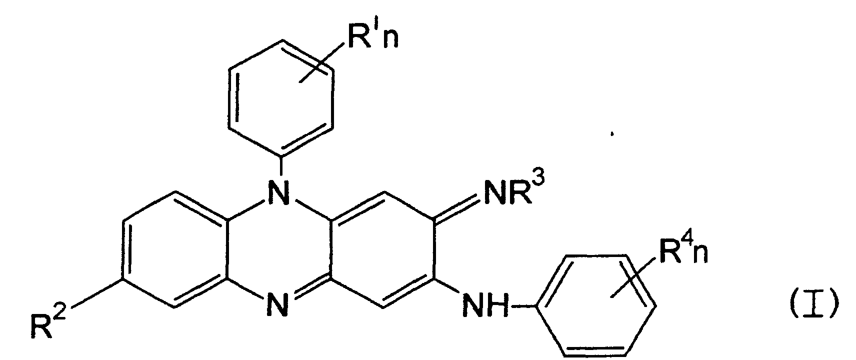 dans laquelle :
R¹ et R⁴ sont les mêmes et chacun est un atome d'halogène, ou un radical alkyl-(C₁-C₄), alkoxy-(C₁-C₄), fluoroalkoxy-(C₁-C₄) ou haloalkyl,
R² est un atome d'hydrogène ou d'halogène,
R³ est un radical 4-tétraméthylpipéridyl et n est 1, 2 ou 3 à condition que (a) lorsque n est 2 ou 3, (R¹)n et (R⁴)n ne soient pas le 3,4-dichloro et que (b) lorsque n est 1, les substituants R¹ et R⁴ ne puissent pas représenter les radicaux alkyl-(C₁-C₄) ou alkoxy-(C₁-C₄) ou un halogène en position 4.

3. L'utilisation de la riminophénazine telle que revendiquée dans la revendication 1, dans laquelle ladite riminophénazine est de la formule générale suivante (I) 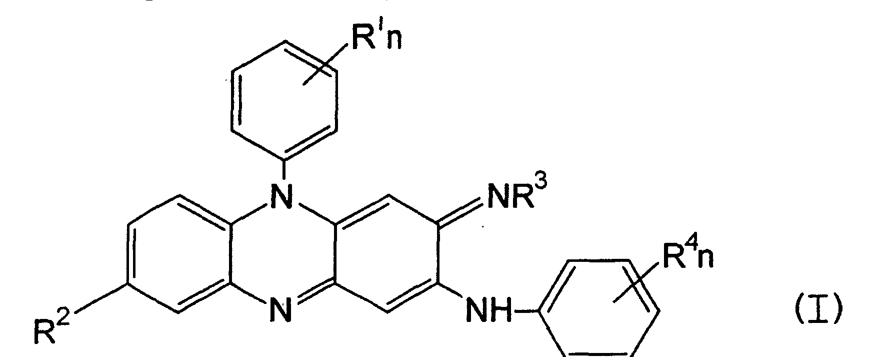 dans laquelle
R¹ et R⁴ sont les mêmes et chacun est un atome d'hydrogène, un atome d'halogène ou un radical alkyl,
R² est un atome d'hydrogène ou d'halogène,
R³ est sélectionné parmi l'hydrogène, l'alkyle, l'alkyle substitué, le cycloalkyle, le cycloalkylalkyle, ou est un radical hétérocyclique ou hétérocyclique-alkyl, et n est 1, 2 ou 3.

4. L'utilisation telle que revendiquée dans la revendication 2 d'une riminophénazine de formule générale I, dans laquelle R¹ et R⁴ sont les mêmes et chacun est un radical trifluorométhyl, R² est un atome d'hydrogène ou d'halogène, R³ est un 4-tétraméthylpipéridyl, n est 1, 2 ou 3.

5. L'utilisation telle que revendiquée dans la revendication 2 ou la revendication 4 dans laquelle R³ est un radical 4-(2,2,6,6,-tétraméthylpipéridyl).

6. L'utilisation telle que revendiquée dans la revendication 2, dans laquelle les radicaux haloalkyl R¹ et R⁴ sont des radicaux trifluorométhyl.

7. L'utilisation d'une riminophénazine telle que revendiquée dans la revendication 3, dans laquelle R¹ et R⁴ sont le 4-chloro, R² est de l'hydrogène, R³ est de l'isopropyle et n est 1, c.-à-d. le composé clofazimine.

8. L'utilisation telle que revendiquée dans les revendications 2 et 4 d'un composé de formule (I), dans laquelle n est 1, R¹ et R⁴ sont chacun un radical trifluorométhyl en position 4, R² est de l'hydrogène et R³ est un radical 4-(2,2,6,6,-tétraméthylpipéridyl).

9. L'utilisation telle que revendiquée dans la revendication 2 d'un composé de formule (I), dans laquelle n est 2, R¹ et R⁴ sont identiques et sont des atomes de chlore en positions 2,4, 3,4 ou 3,5, R² est de l'hydrogène et R³ est un radical 4-(2,2,6,6-tétraméthylpipéridyl).

10. L'utilisation telle que revendiquée dans la revendication 2 d'un composé selon la formule (I), dans laquelle n est 3, R¹ et R⁴ sont des atomes de chlore en position 3, 4 et 5, R² est de l'hydrogène et R³ est un radical 4-(2,2,6,6,-tétraméthylpipéridyl).

11. Un composé de formule générale (I) 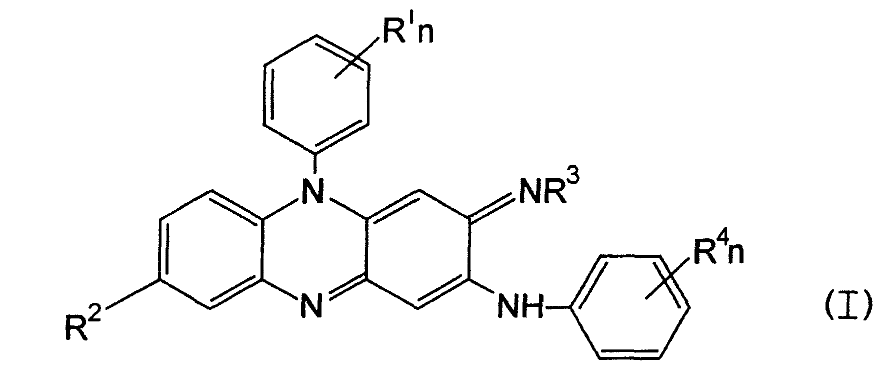 dans laquelle
R¹ et R⁴ sont les mêmes et son sélectionnés parmi l'halogène, l'alkyl-(C₁-C₄), l'alkoxy-(C₁-C₄), les radicaux fluoroalkoxy-(C₁-C₄) et trifluorométhyl, n est 1, 2 ou 3, R³ est un radical 4-(2,2,6,6-tétraméthylpipéridyl) et R² est sélectionné parmi l'hydrogène et l'halogène à condition que (a) lorsque n est 2 ou 3, (R¹)n et (R⁴)n ne soient pas 3,4-dichloro et que (b) lorsque n = 1, les substituants R¹ et R⁴ ne puissent pas représenter les radicaux alkyl-(C₁-C₄) ou alkoxy-(C₁-C₄) ou un halogène en position 4.

12. Un composé selon la revendication 11, dans lequel les cycles phényles contenant (R¹)n et (R⁴)n sont des cycles 3,5-dichlorophényles, R² est de l'hydrogène et R³ est un radical 4-(2,2,6,6,-tétraméthylpipéridyl), c.-à-d. le composé N,5-*bis*(3,5-dichlorophényl)-3,5-dihydro-3-[(2',2',6',6'-tétraméthylpipéridyl)imino]-2-phénazinamine.

13. Un composé selon la revendication 11, dans lequel les cycles phényles contenant (R¹)n et (R⁴)n sont des cycles 2,4-dichlorophényles, R² est de l'hydrogène et R³ est un radical 4-(2,2,6,6-tétraméthylpipéridyl), c.-à-d. le composé N,5-bis(2,4-dichlorophényl)-3,5-dihydro-3-[(2',2',6',6'-tétraméthylpipéridyl)imino]-2-phénazinamine.

14. Un composé selon la revendication 11, dans lequel R¹ et R⁴ sont des atomes de chlore en positions 3, 4 et 5, R² est de l'hydrogène et R³ est un radical 4-(2,2,6,6-tétraméthylpipéridyl), c.-à-d. le composé N,5-*bis*(3,4,5-trichlorophényl)-3,5-dihydro-3-[(2',2',6',6'-tétraméthylpipéridyl)imino]-2-phénazinamine.

15. Un composé selon la revendication 11, dans lequel les cycles phényles contenant (R¹)n et (R⁴)n sont des cycles 3-trifluorométhyl-4-chlorophényles, R² est de l'hydrogène et R³ est un radical 4-(2,2,6,6-tétraméthylpipéridyl), c.-à-d. le composé N,5-*bis*(3-trifluorométhyl-4-chlorophényl)-3,5-dihydro-3-[(2',2',6',6'-tétraméthylpipéridyl)imino]-2-phénazinamine.

16. Un composé selon la revendication 11, lequel est N,5-*bis*(4-trifluorométhylphényl)-3,5-dihydro-3-[(2',2',6',6'-tétraméthylpipéridyl)imino]-2-phénazinamine.

17. Un composé selon la revendication 11, lequel est N,5-*bis*(3-trifluorométhylphényl)-3,5-dihydro-3-[(2',2',6'-tétraméthyl-4-pipéridyl)imino]-2-phénazinamine.

18. Un composé selon la revendication 11, lequel est N,5-*bis*(4-trifluorométhoxyphényl)-3,5-dihydro-3-[(2',2',6',6'-tétraméthyl-4-pipéridyl)imino]-2-phénazinamine.

19. Un composé selon la revendication 11, lequel est N,5-*bis*(3-trifluorométhylphényl)-8-chloro-3,5-dihydro-3-[(2',2',6',6'-tétraméthyl-4-pipéridyl)imino]-2-phénazinamine.

20. Un composé de la formule (I) 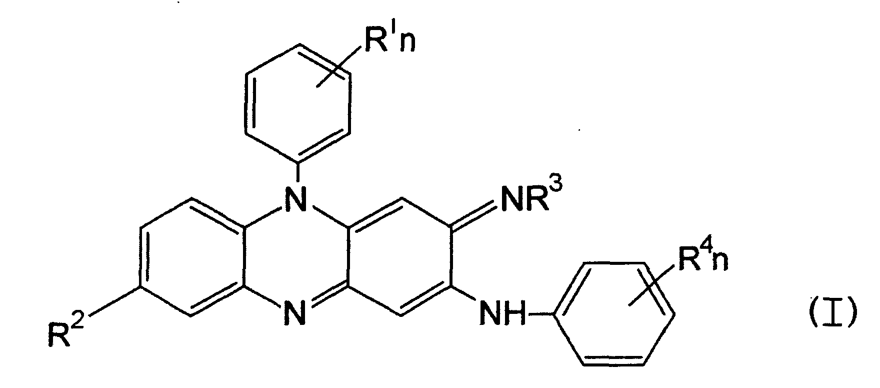 dans laquelle le composé est sélectionné dans le groupe constitué
(a) du composé B4123 c.-à-d. N,5-bis(3-chlorophényl)-8-chloro-3-[(2',2',6',6'-tétraméthyl-4-pipéridyl)imino]-2-phénazinamine,
(b) du composé B4158 c.-à-d. N,5-*bis*(4-isopropylphényl)-3,5-dihydro-3-[(2',2',6',6'-tétraméthyl-4-pipéridyl)imino]-2-phénazinamine,
(c) du composé B4159 c.-à-d. N,5-*bis*(4-isopropylphényl)-8-chloro-3,5-dihydro-3-[(2',2',6',6'-tétraméthyl-4-pipéridyl)imino]-2-phénazinamine et
(d) du composé B4174 c.-à-d. N,5-*bis*(4-méthoxyphényl)-3,5-dihydro-3-[(2',2',6',6'-tétraméthyl-4-pipéridyl)imino]-2-phénazinamine.

21. Une composition destinée au traitement d'un patient cancéreux qui a développé, ou pourrait développer, une résistance à une substance thérapeutiquement active utilisée dans le traitement du cancer, ladite composition contenant un composé tel que revendiqué dans n'importe lesquelles des revendications 11 à 20 et un support acceptable d'un point de vue pharmaceutique.

22. Une composition telle que revendiquée dans la revendication 21, dans laquelle la composition contient aussi un composé connu pour le traitement du cancer, lequel n'est pas une riminophénazine.

23. Un procédé destiné à la préparation d'un composé de la formule générale (I) 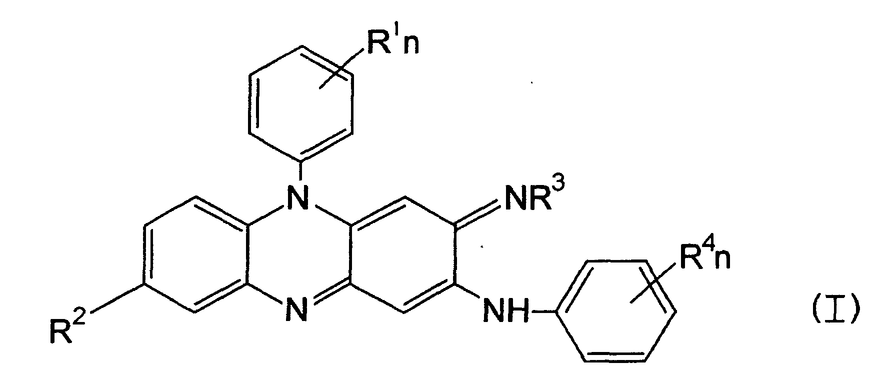 dans laquelle
R¹, R², R³, R⁴ et n ont les significations définies dans la revendication 11,
qui consiste à faire réagir un composé de formule (IV) 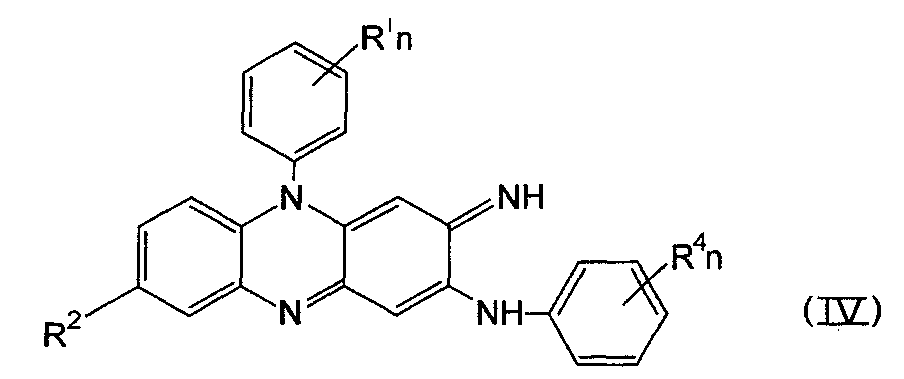 dans laquelle
avec un composé de formule R³-NH₂
dans laquelle R¹, R², R⁴ et n sont tels que définis ci-dessus.
